# EUROPEAN PATENT SPECIFICATION

(11) **EP 1 781 377 B1**
(45) Date of publication and mention of the grant of the patent: **11.04.2012**
(21) Application number: 05764348.8
(22) Date of filing: 04.08.2005
(51) Int. Cl.: A61P 35/02, A61K 38/07, A61K 35/55

(54) **ELASTASE INHIBITOR IN LEUKEMIA**
ELASTASEHEMMER BEI LEUKÄMIE
INHIBITEUR DE L'ELASTASE UTILISE DANS LE TRAITEMENT DE LA LEUCEMIE

(30) Priority: 10.08.2004 IL 16345304
(43) Date of publication of application: 09.05.2007
(73) Proprietor: YEDA RESEARCH AND DEVELOPMENT CO., LTD., 76100 Rehovot (IL)
(72) Inventor: LAPIDOT, Tsvee, 74046 Ness Ziona (IL); TAVOR, Sigal, 63346 Tel Aviv (IL); PETIT, Isabelle, F-77150 Lesigny (FR)
(74) Representative: Grünecker, Kinkeldey, Stockmair & Schwanhäusser
(86) International application number: PCT/IL2005/000840
(87) International publication number: WO 2006/016353

(56) References cited:
- EP-A- 0 289 336
- EP-A- 0 525 973
- EP-A- 0 630 382
- WO-A-99/12934
- WO-A-03/079969
- WO-A-2004/041115
- US-A- 6 103 498
- TAVOR S ET AL: "HUMAN AML CELLS SECRETE ELASTASE THAT REGULATES THEIR SDF-1/CXCR4 DEPENDENT HOMING INTO THE BONE MARROW OF TRANSPLANTED NOD/SCID B2MNULL MICE" BLOOD, W.B.SAUNDERS COMPANY, ORLANDO, FL, US, vol. 100, no. 11, 16 November 2001 (2001-11-16), page 750A, XP001194624 ISSN: 0006-4971

## Description

### FIELD OF THE INVENTION

The invention relates to the use of an elastase inhibitor in the treatment of human leukemia, such as acute myelogenous (AML), with an elastase inhibitor.

### BACKGROUND OF THE INVENTION

Stem cells are capable of self renewal and division, leading to more stem cells and to differentiated cells. Hematopoietic stem cells (HSC) or hematopoietic progenitor cells (HPC) have the property of giving rise to sufficient hematopoietic activity to rescue a lethally irradiated recipient from hematopoietic failure (Morrison et al. 1995).

Bone marrow contains mesenchymal and hematopoie5.19tic stem cells. The mesenchymal stem cells give rise to adipocytic, chondrocytic and osteocytic lineage, including the stromal cells of bone marrow (Pittenger et al. 1999). The hematopoietic stem cells (HSCs) have been found to give rise to lymphoid, myeloid and erythrocytic lineages.

In mouse, HSCs represent a rare population of 0.01% of whole bone marrow and have been isolated using the combination of markers: Thy^{low} Lineage- Scal+ c-kit^{high} (KTLS). In humans CD34+ Thy-1+ Lin- hematopoietic stem cells are the human equivalents of the mouse KTLS hematopoietic stem cells (Ikuta et al 1992).

The mechanisms that guide circulating hematopoietic progenitor cells (HPC or HSC) are clinically significant because the success of stem cell transplantation depends on efficient targeting of grafted cells in a recipient's bone marrow (Mazo and von Adrian 1999). It is due to this targeting (or homing) of transplanted cells that bone marrow transplantations can be performed by simple intravenous infusion, rather than requiring invasive surgery, as in the case with the transplantation of any other organ. Homing of HPC can be defined as the set of molecular interactions that allow circulating HPC to recognize, adhere to, and migrate across bone marrow endothelial cells and results in the accumulation of HPC in the unique hematopoiesis-promoting microenvironment of the bone marrow. Homing of progenitor cells can be conceived as a multi-step phenomenon. HPC arriving to the bone marrow must first interact with the luminal surface of the bone marrow endothelium. This interaction must occur within seconds after the HPC has entered the bone marrow microvasculature and provide sufficient mechanical strength to permit the adherent cell to withstand the shear force exerted by the flowing blood. Adherent HPC must then pass through the endothelial layer to enter the hematopoietic compartment. After extravasation, HPC encounter specialized stromal cells whose juxtaposition supports maintenance of the immature pool of cells by self-renewal process in addition to lineage-specific HPC differentiation, proliferation and maturation, a process that involves stroma-derived cytokines and other growth signals.

SDF-1, also called pre-B-cell growth-stimulating factor (PBSF), has been reported to be a powerful chemo attractant (chemokine) for lymphocytes, monocytes, and primary CD34+ cells. SDF-1 is a chemotactic factor that induces migration of cells and the direction of cell movement is determined by the concentration gradient of SDF-1 (Kim and Broxmeyer 1998), low in the peripheral blood and high in the bone marrow. Since SDF-1 is produced by bone marrow stroma cells, it was hypothesized that an SDF-1 gradient is formed between the bone marrow microenvironment to the blood system. This gradient attracts HPC, and retains them in the bone marrow microenvironment, unless, this gradient is broken by administered or induced effectors molecules in the blood.

The receptor of SDF-1, CXCR4, is expressed on many cell types, including bone marrow cells, mobilized bone marrow cells, cord blood cells, including the sub population of cord blood CD34+ cells, CD34+CD38- cells, which are pluripotent hematopoietic precursor cells. Treatment of the human HPCs, CD34+ cells, with anti CXCR4 antibody before transplantation results in inhibition of bone marrow engraftment in transplanted NOD/SCID mice (Peled et al Science 1999).

Immature human CD34+ cells and primitive CD34+/CD38-/low cells, which do not migrate toward a gradient of SDF-1 in vitro, and do not home and repopulate in vivo the murine bone marrow, can become functional repopulating cells by short-term 16 to 48 hr in vitro stimulation with cytokines such as SLF and IL-6 prior to transplantation (Kollet et al. 2000, Peled et al. 1999 Lapidot 2001). These cytokines increase surface CXCR4 expression, migration toward SDF-1 in vitro, homing and repopulation in vivo.

It has been reported that SDF-1 is also a key factor in stimulation of human stem cell adherence to endothelial cells in the bone marrow microvasculature (Peled et al The Journal of Clinical Investigation 1999). Therefore, SDF-1 is implicated not only as chemo attractant for stem and progenitor cells, but also as mediator of integrin dependent cell adhesion and transendothelial migration required for engraftment in the bone marrow.

Throughout adult life, the hematopoietic system is maintained by constant production of mature lymphoid, myeloid and erythroid cells, which are released from the bone marrow (BM) to the periphery and to secondary organs. Interestingly, hematopoietic stem cells, which mainly reside within the BM, are also found circulating in the blood at very low levels. This egress of adequate mature and immature cells must be tightly regulated, but the molecular mechanisms controlling migration and cell egress are largely uncharacterized.

HPCs can be mobilized from the bone marrow to the peripheral blood in response to injected cytokines such granulocyte-macrophage colony-stimulating factor (GM-CSF), granulocyte colony-stimulating factor (G-CSF), and Steel factor (SLF) [Siena et al, 1989, Duhrsen et al 1988, Drize et al 1996]. Mobilization of stem cells from donor's bone marrow into the blood and their retrieval from the blood, for transplantation procedures, is increasingly being used world wide, and is replacing the recovery of these stem cells from the donor's bone marrow using invasive surgery.

Mobilization allows bone marrow repopulation with own HSC, recovered and reserved from patients prior irradiation and chemotherapic treatments (autologous transplantation). The recovery of HSC is greater from mobilization than from cord blood or from bone marrow surgery.

Studies aimed at deciphering the mechanism of stem and progenitor cells egress of BM cells have focused on leukocytosis induced by various stress inducing agents such as LPS, chemokines or cytokines, including granulocyte colony-stimulating factor (G-CSF), which is widely used to mobilize and harvest HSC for clinical transplantation [To, 1997][Thomas, 2002]. In recent years; the mechanism governing G-CSF induced moblization has began to emerge: G-CSF induces expansion of myeloid cells which release large amounts of neutrophil proteases such as elastase and cathepsin G in the BM that degrade stromal VCAM-1, ICAM-1, the P-selectin receptor PSGL-1 as well as the chemokine SDF-1 and the cytokine kit ligand [Lapidot, 2002][Papayannopoulou, 2004]. Both VCAM-1/VLA-4, c-kit/kit ligand and SDF-1/CXCR4 interactions are believed to be crucial regulators of hematopoietic cell anchorage and retention within the BM. In addition, metalloproteinases such as MMP-9 also participate in G-CSF induced release of cells [Heissig, 2002]. Interestingly, heterozyous germline mutations in the ELA2 gene encoding the human leukocyte elastase have been associated with several inherited neutropenic syndromes such as cyclic neutropenia and Kostman disease, which are characterized by a severe impairment in neutrophil release into the circulation [Aprikyan, 2001].

Neutrophil - or leukocyte- elastase is a serine protease stored in azurophilic granules of myeloid cells and is released upon activation and degranulation. Elastase is a very broad range proteolytic enzyme, its substrates include various extracellular matrix proteins, such as elastin, fibronectin and collagen as well as adhesive molecules like ICAM-1 and junctional cadherins, suggesting a role for elastase in facilitating cell transendothelial migration [Ginzberg, 2001]. In addition, elastase degrades numerous soluble proteins like coagulation factors, immunoglobulins, complement, protease inhibitors, cytokines, growth factor and their receptors [Bank, 2001] [Lee, 2001].

Clinical and laboratory features of leukemia are caused by suppression of normal blood cell formation and organ infiltration by the malignant clone. Inhibitory factors produced by leukemic cells or replacement of marrow space may suppress normal hematopoiesis, with ensuing anemia, thrombocytopenia, and granulocytopenia. Organ infiltration of leukemia cells results in enlargement of the liver, spleen, and lymph nodes, with occasional kidney and gonadal involvement. Meningeal infiltration results in clinical features associated with increasing intracranial pressure (e.g., cranial nerve palsies).

Leukemias were originally termed acute or chronic based on life expectancy but now are classified according to cellular maturity. Acute leukemia consists of predominantly immature cells (usually blast forms); chronic leukemia, more mature cells.

Acute leukemias are divided into lymphoblastic (ALL) and myelogenous (AML) types, which may be further subdivided by morphologic and cytochemical appearance according to the French-American-British (FAB) classification (Table A) or immunophenotype. The specific B-cell and T-cell and myeloid-antigen monoclonal antibodies, together with flow cytometry, are very helpful for classifying ALL versus AML, which is critical for treatment.

EP0525973 discloses elastase inhibitors for treating inflammatory diseases and proteinase 3 (PR-3) inhibotors for treating cancer.

WO99/12934 discloses compounds acting as neutrophil elastase inhibitors. WO03/079969 is related to the use of elastase inhibitors in the context of necrosis.

EP0630382 discloses inhibitors of human leukocyte elastase (HLE) and human neutrophil elastase for the treatment of inflammatory disorders.

Tavor, S. et al. (Blood 100 (2001), p. 750A (Annual Meeting Abstract #2969)) discloses that acute myelogenous leukemic (AML) cells secrete high levels of elastase into the serum of patients afflicted with AML and that elastase regulates the SDF-1 dependent homing of AML cells into the bone marrow.

| **FAB Classification** | **Description** | **FAB Classification** | **Description** |
|---|---|---|---|
| **Acute fymphoblastic leukemia** | | **Acute myelogenous leukemia** | |
| L1 | Lymphoblasts with uniform, round nuclei and scant cytoplasm | M1 | Undifferentiated myeloblastic; no cytoplasmic granulation |
| | | M2 | Differentiated myeloblastic; few to many cells may have sparse granulation |
| L2 | More variability of lymphoblasts: nuclei may be Irregular with more cytoplasm than L1 | | |
| | | M3 | Promyelocytic; granulation typical of promyelocytic morphology |
| L3 | Lymphoblasts have finer nuclear chromatin and blue to deep blue cytoplasm with cytoplasmic vacuolization | | |
| | | M4 | Myelomonoblastic; mixed myeloblastic and monocytoid morphology |
| | | M5 | Monoblastic; pure monoblastic morphology |
| | | M6 | Erythroleukemic; predominantly immature erythroblastic morphology, sometimes megaloblastic appearance |
| | | M7 | Megakaryoblastic; cells have shaggy borders that may show some budding |

Chronic leukemia is described as lymphocytic (CLL) or myelocytic (CML).

Myelodysplastic syndromes represent progressive bone marrow failure, but with an insufficient proportion of blast cells (< 30%) for definite diagnosis of AML; 40 to 60% of cases evolve into AML.

Acute myeloblastic leukemia (AML) is characterized by uncontrolled proliferation within the BM of malignant myeloid progenitors arrested in their maturation process and the egress of these abnormal cells into the circulation. Previous studies reported high levels of intracellular elastase activity and secretion of elastase protein by AML cells [Hunter, 2003].

Leukemic cells appear to express high level of several proteases such as MMP-2, MMP-9, MT1-MMP (Ries et al Clinical cancer research 1999, (5) 1115) and elastase.

Evidence recently emerged that elastase has a role in the development of chronic myeloid leukemia associated with the Philadelphia chromosome and in patients under chronic phase (CML-CP) only [El-Ouriaghli, Blood 15 volume 102 number 10, 2003]. El-Ouriaghli reports that both CML cell proliferation and normal progenitor cell (NPC) proliferation are inhibited by elastase, however CML proliferation is inhibited at a lesser extent.

G-CSF is a growth factor inducing proliferation of NPC's. According to El-Ouriaghli, elastase does not inhibit proliferation directly but it does it by digesting G-CSF, and the G-CSF receptor (Hunter et al 2003) and resulting in NPC's growth reduction. El-Ouriaghi explains that, since CML cell's growth is less dependent on exgenously transmitted growth factors compared to NPC's, CML is less affected by elastase, but not completely insensitive to it.

El-Ouriaghli indicates that sustained concentration of different proteases such as MM-9, neutrophil serine proteinase such as proteinase 3, or cathepsin G and including elastase in CML could be responsible for inducing characteristic immature marrow cells into the blood of CML patients.

SDF-1/CXCR4 interactions are crucial for homing and repopulation of normal human stem cells transplanted into immundeficient NOD/SCID mice [Kollet, 2001][Peled, 1999]. We recently showed that malignant human AML and pre B ALL cell homing to the BM and spleen NOD/SCID/B2mnull mice is also CXCR4 dependent [Tavor, 2004, Asaf, Blood].

The translocation that is present in more than 90% of patients with acute promyelocytic leukemia (APL) creates two fusion proteins PML-RAR and RAR-PML. The proteolytic processing by elastase was recently suggested to play an important role in the development of APL. Lane et al (Cell vol 115 305, 2003) showed that the fusion protein PML-RAR associated with acute promyelocytic leukemia (APL) is cleaved by neutrophil elastase and that neutrophil elastase deficient mice are partially protected from development of APL (Lane, 2003).

More recently, the use of elastase was proposed for treatment of leukemia to induce leukemia reactive cytotoxic T cells (Fujiwara et al. , 2004 Blood, 103 (5) 3076).

Thus, there exists a need to provide a feasible therapy method for treating leukemia.

### SUMMARY OF THE INVENTION

The invention relates to the use of an elastase inhibitor in the manufacture of a medicament for the treatment of leukemia, preferably acute leukemia and more preferably for the treatment of AML.

In one embodiment of the invention, elastase inhibitor is an elastase-neutralizing antibody, MeOSuc-AAPV-CMK or al-antitrypsin.

The present disclosure relates to the use of an elastase inhibitor, and optionally a mobilizing agent, in the manufacture of a medicament for assisting hematopoietic stem cell (HSC) autotransplantation in patients suffering from leukemia such as acute leukemia and preferably AML.

In a further embodiment, the invention relates to the use of an elastase inhibitor in the manufacture of a medicament for preventing or inhibiting egress of leukemic cells, from hematopoietic organs, preferably the bone marrow, to the blood in patients suffering of leukemia such as acute leukemia and preferably AML.

The present disclosure relates to the use of an elastase inhibitor in the manufacture of a medicament for preventing or inhibiting migration of leukemic cells.

In a another further embodiment, the invention relates to the use of an elastase inhibitor in the manufacture of a medicament for preventing or inhibiting proliferation of leukemic cells, such as acute leukemia and preferably AML.

The present disclosure relates to the use of an elastase inhibitor for separating normal hematopoietic stem cells from leukemic cells in a mixture of cells extracted from a patient, comprising the step of incubating the mixture of cells with an elastase inhibitor and collecting fast migrating cells, preferably cells migrating to an SDF-1 gradient.

In a another further embodiment, the invention relates to an elastase inhibitor for use in a method of treatment of leukemia,

In another further embodiment of this aspect, the invention relates to acute leukemia and preferably AML.

In another further embodiment of this aspect, the invention relates to an elastase inhibitor such as an elastase-neutralizing antibody; MeOSuc-AAPV-CMK.and al-antitrypsin for use in a method of treating leukemia.

Also disclosed is a method of hematopoietic stem cell (HSC) autotransplantation in a patient suffering of leukemia comprising the steps of administrating a mobilizing agent and contacting the mobilized cells with an elastase inhibitor collecting the cells and transplanting back the cell into the patient. Preferably, the step of contacting the elastase inhibitor with the mobilized cells is carried out ex-vivo, prior to transplanting back the cell into the patient.

In another further embodiment, the invention relates to an elastase inhibitor for use in a method of treatment of a patient suffering of leukemia.

In another further embodiment, the invention relates to an elastase inhibitor for use in a method of preventing or inhibiting egress of leukemic cells, from an hematopoietic organ to the blood, in a patient suffering of leukemia.

Also disclosed is a method of diagnosing the maturation stage of leukemic cell in a patient suffering of acute leukemia comprising the step of monitoring the level of elastase in the bone marrow of said patient.

Also disclosed is a method for separating a mixture of normal hematopoietic stem cells and leukemic cells extracted from a patient, comprising the step of incubating the mixture of cells with an elastase inhibitor, and collecting fast migrating cells, optionally migrating trough an SDF-1 gradient

The present disclosure further relates to an animal model for assessing egress of leukemic cells, comprising injecting an immunodefficient mouse with human leukemic and allowing the cells to engraft the bone marrow to obtain a chimeric mouse.

The present disclosure further relates to a method for isolating a drug capable of inhibiting or preventing leukemic cell egress or growth comprising the use of the animal model of the invention.

The present disclosure further relates to a drug capable of inhibiting or preventing leukemic cell egress or proliferation obtainable using the animal model of the invention.

The present disclosure further relates to a cell preparation obtainable using the method of separation of the invention.

The present invention is further described by the appended claims.

### BRIEF DESCRIPTION OF THE FIGURES

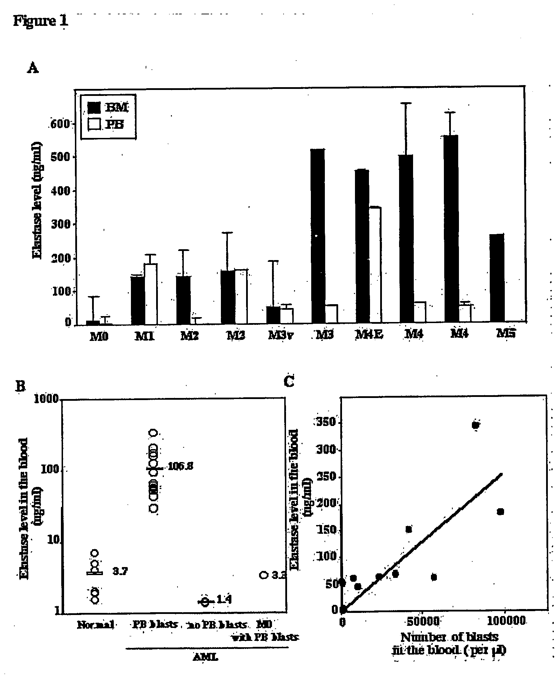

Figure 1 shows levels of elastase in BM and peripheral blood of AML patients. (A) Plasma levels of elastase from BM (black bars) and peripheral blood (white bars) from ten AML patients with variable phenotype were determined by ELISA. Each sample was measured in triplicate and data shown represent average ± standard error (SE). (B) Plasma levels of elastase in the peripheral blood from normal individuals (Normal), AML patients displaying blasts in the periferal blood (PB), without blasts in the circulation (no PB blasts) or AML patient with M0 FAB subtype with blasts in PB. Each sample was measured in duplicate or triplicate and data shown represent average. Average of each group is indicated. (C) Correlation between PB elastase levels and number of circulating AML blasts. Plasma levels of elastase were plotted against the number of circulating blasts. R=0.6, p=0.01

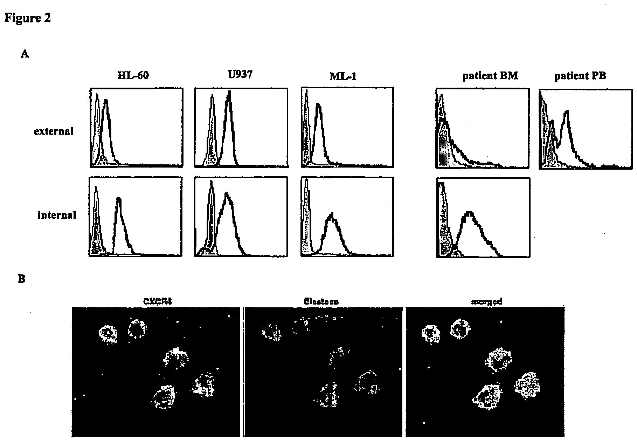

**Figure 2** shows cell surface expression of elastase on AML cells. (A) AML cell lines (HL-60, U937 and ML-1) and primary AML cells from BM (patient BM) and PB (patient PB) were stained for external and internal elastase. FACS analyses of AML cell lines (left panel) and primary leukemic cells (right panel) of elastase. The shaded histograms show staining with isotype-matched control antibodies, the open histograms show staining with elastase antibodies. (B) Immunocytochemical analysis of membranal elastase localization in cells obtained from primary AML patient adhered to poly-L-lysine and indirectly immunolabeled with anti-CXCR4 and anti-elstase Abs. Original magnification 100X.

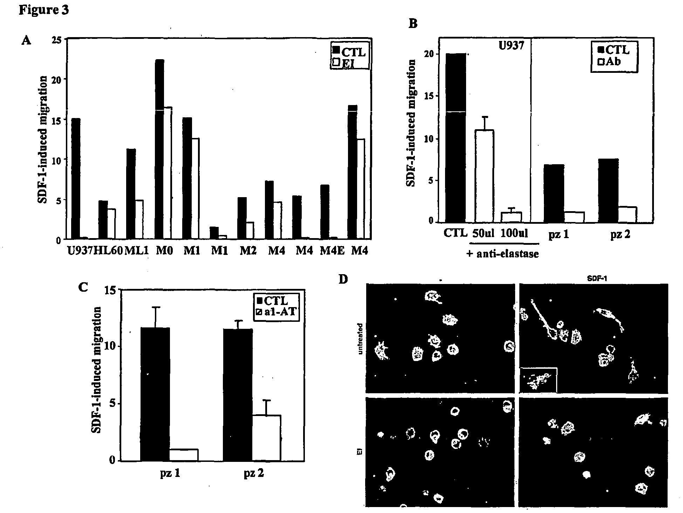

**Figure 3** shows that elastase secretion by AML cells affect their SDF-1-induced transwell migration. (A) In vitro transwell migration assay of AML cells, either untreated or treated for 30 minutes with elastase inhibitor, 10µg/ml EI (MeOSuc-AAPV-CMK) (A), anti elastase Abs (B) or a1-antitrypsin (a1-AT) (C). The results show the percentage of migrated cells toward 125 ng/ml SDF-1 after 4 hours. (D) Immunocytochemical analysis of primary AML cells on poly-L-lysine coated surfaces. Cells were pre-treated for 30 minutes with elastase inhibitor (EI) or left untreated, allowed to adhere in the absence (-) or presence of 200 ng/ml SDF-1, fixed and indirectly immunolabeled with anti-CXCR4 and anti-elastase Ab. Original magnification 100X.

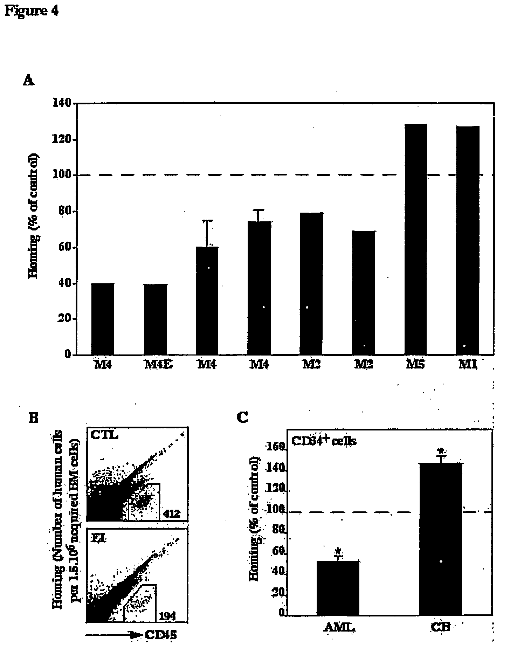

**Figure 4** shows that elastase inhibitor prevents homing of AML cells. (A) Primary human AML cells from 8 patients either untreated or after 30 minutes incubation with elastase inhibitor (10 µg/ml) were injected into sublethally irradiated B2mnull NOD/SCID mice. Percentages of human CD45+ cells in the BM were determined after 16 hours. (A) Data show the percentage of elastase inhibitor -treated human cells as compared to untreated control (100%). (B) FACS analysis of a representative experiment. Numbers shown represent the number of human CD45+ cells per 1.5x10⁶ acquired BM cells. (C) Comparison of the effect of elastase inhibitor (10 µg/ml) on the homing of primary AML CD34+ cells and normal CB enriched CD34+ cells.

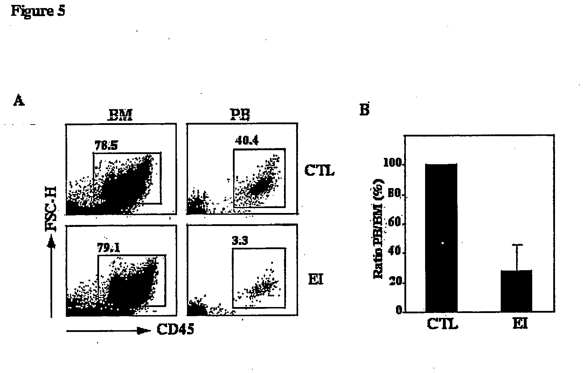

**Figure 5** shows that inhibition of elastase prevents the egress of engrafted human AML cell into the circulation. Engrafted NOD/SCID mice with AML cells were treated with 1mg elastase inhibitor for 4 consecutive days. Levels of AML cells in the BM and in the peripheral blood were determined by CD45 staining. FACS analysis from one representative experiment is shown (A). Panel B show the percentage of human engrafted cells in the PB / percentage of engrafted cells in the BM with or without treatment of elastase inhibitor. Due to variability in percentage of engraftment among mice, comparison of the ratio PB/BM was performed. The data represents the average results from 4 independent experiments

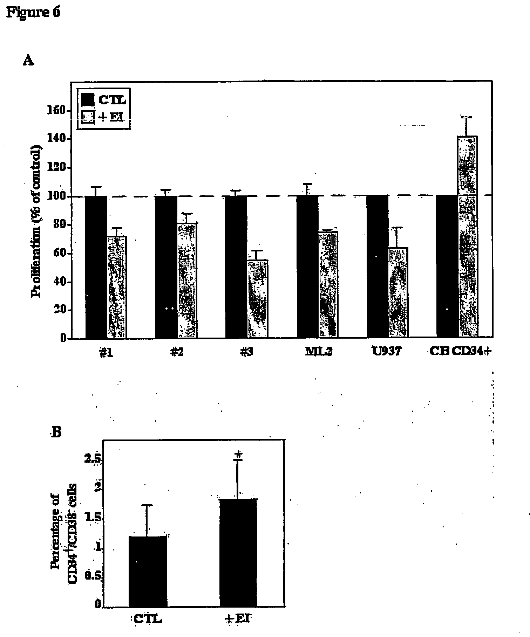

**Figure 6** shows that elastase inhibitor inhibits the proliferation of AML cells. Primary AML cells, AML cell lines and human cord blood (CB) cells with or without 10 µg/ml elastase inhibitor (EI), were cultured for 3-7 days and the number of viable cells was determined using trypan blue exclusion (A). Enriched CD34+ CB cells from full term deliveries were grown in RPMI/10%FCS with SCF, FLT3L and IL6. The percentage of CD34+/38- cells from the total enriched CD34+ CB cells cultured with and without EI was determined by FACS (B).

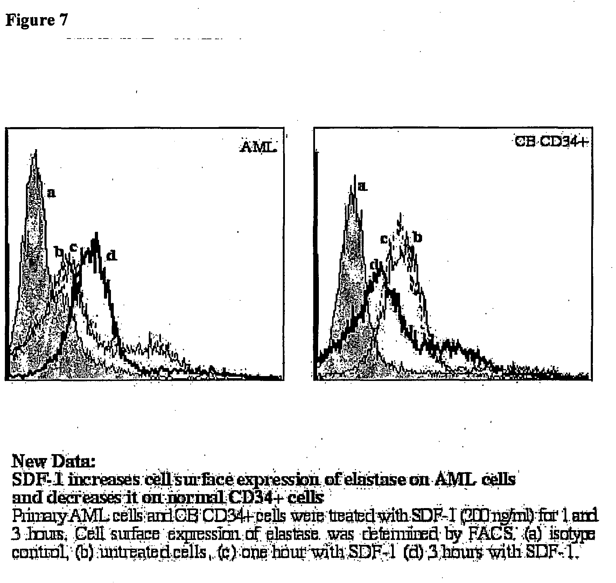

**Figure 7** shows that SDF-1 increases cell surface expression of elastase on AML cells and decreases it on normal CD34+ cells. Primary AML cells and CB CD34+ cells were treated with SDF-1 (200ng/ml) for 1 and 3 hours, and expression of cell surface of elastase was determined by FACS(a) isotype control, (b) untreated cells, (c) one hour with SDF-1, (d) 3 hours with SDF-1.

### DETAILED DESCRIPTION OF THE INVENTION

The invention relates to the use of an elastase inhibitor for the treatment of leukemia.

The present disclosure relates to the use of an elastase inhibitor for inhibiting proliferation of leukemic cells along with enhancing the growth of normal stem cells and particularly a lineage of stem cells more suitable for transplantation..

In another aspect, the invention relates to the use of an elastase inhibitor for preventing migration and egress of leukemic cells from hematopoietic organs of a patient, such as e.g. the bone marrow, into the circulation, and an assay for screening of drugs capable to inhibit leukemic cell egress is also disclosed

The present disclosure further relates to the use of an elastase inhibitor for assisting autologous transplantation in a leukemic patient.

In addition, the disclosure relates to diagnostic methods for detecting maturation stage of leukemic cells in leukemic patients.

The invention therefore further relates to the use of an elastase inhibitor, for manufacture of a medicament for the treatment of leukemia.

The invention relates to the use of an elastase inhibitor in the treatment of any kind of leukemia, such as chronic leukemia and preferably acute leukemia, such as acute acute promyelocytic leukemia (APL) and acute myelogenous (AML).

The invention is based on the following *in vitro* experimental findings:
AML cells not only secrete elastase independently of external stimulus, but also constitutively express elastase homogeneously assembled on the cell surface. Cepinskas et al.(1999) indicated that in activated neutophils, membrane bound elastase is catalytic active and resistant to proteolyses by circulating proteases, in contrast to soluble elastase. Thus AML's membrane elastase is protected from proteolyses by circulating proteases, and may allow AML cells to penetrate the bone marrow ECM (extra cellular matrix) and endothelial barriers and facilitate their dissemination into the circulation. The results obtained in vitro show that the role of the membrane bound elastase is not only assisting AML penetration trough mechanical barriers such as the BM endothelium but, as observed in an in vitro migration assay using three different elastase inhibitors MeOSuc-AAPV-CMK (EI), al anti trypsin or anti elastase antibody, elastase is required for AML motility (both directional, SDF-1 dependent, and spontaneous migration) e.g. by inducing cytoskeletal rearrangements and cell polarization.

It was recently shown that homing of malignant human AML and pre B ALL cell to the BM and spleen in NOD/SCID/B2mnull mice is also dependent on SDF-1/CXCR4 interactions [Tavor, 2004, Asaf, Blood]. In view of the above results showing the role of elastase in AML migration, it was assumed that SDF-1 may have a role in elastase regulation. Indeed, it was found in accordance with the present invention that SDF-1 regulates expression of cell surface elastase and has opposite effect in AML versus in normal CB CD34+ cells. While SDF-1 increased surface elastase in AML cells, it decreases surface elastase on normal CB CD34+ enriched cells.

AML is characterized by extensive and uncontrolled AML cell proliferation within the BM. Therefore, we checked the possibility that elastase may be involved also in regulation of AML cell proliferation.

To test the role of elastase in AML proliferation, AML cells (primary AML cells), or normal cord blood (CB) CD34+ enriched cells, were grown in growth medium with or without an elastase inhibitor (EI) and the number of viable cells was determined after several days in culture. The results observed show that proliferation of AML cells was inhibited by culturing the cells in the presence of elastase inhibitor, while elastase inhibition, enhanced proliferation rate of normal CB CD34+ cultured in the same conditions. Moreover, we found that the percentage of the CD34+ transplantation-competent population, comprising more primitive progenitor cells of the CD34+/38- lineage, was significantly increased after 3 days in culture in the presence of EI, suggesting a role of elastase in differentiation of normal stem cells. Therefore, it is shown here that elastase is necessary for AML proliferation, and that elastase inhibition inhibits AML proliferation and not only increased the proliferation of normal CD34+ cells but in addition, maintained the undifferentiated primitive transplantation-competent CD34+/38- stem cells in the culture.

The invention is based also on the following *in vivo* experimental fmdings using the pre-clinical immune-deficient NOD/SCID mice experimental model, which allows homing and engraftment (or establisment) of human AML stem cells in, and egress out of the bone marrow, thus, mimicking many biological aspects of human AML in patients (Lapidot Nature 1994).

In the experimental model, human (donor) cells e.g. AML cells, are administered to sub-lethally irradiated, non-obese diabetes severe combined immune deficient (NOD/SCID or B2mnull NOD/SCID) mice (recipient), and after a few hours following cell administration (e.g. 16 hours), the human cells reaching or homing to a specific organ (e.g. bone marrow) are monitored (Kollet et al 2001).

The results obtained employing the homing model show that homing of EI pre-treated primary AML cells or enriched CD34+ AML progenitors decreased as compared to untreated cells. Contrary to AML, homing of EI pre-treated normal human CD34+ enriched cells increased compared to untreated cells. Taken together, these results show a central role of elastase in AML homing and an opposite effect of elastase and elastase inhibition on homing in myeloid leukemia CD34+ cells and in normal CD34+ cells.

The chimeric immune-deficient NOD/SCID mice and AML, allowed testing egress of human AML stem cells from the bone marrow to the circulation (egress model). The egress model consists of sublethally irradiated NOD/SCID mice injected with AML cells to establish human AML-mice chimerism (engraftment). AML egress from the bone marrow into the circulation is monitored about two-four weeks after AML injection. Using this AML egress model, it is possible to test the effect of drug administration on AML egress from the bone marrow.

Since our results demonstrated the role of elastase in migration of AML cells, we hypothesized that elastase may be the candidate protease facilitating egress of AML cells from the BM into the circulation. Thus, we tested the effect of EI in egress of AML cells using the egress model. The results obtained show that egress of AML cells (primary and cell line) decreased in EI treated mice compared to non-treated mice (Fig. 5 and table 1 Table B). Thus, these results show that elastase controls human AML egress, and that elastase inhibition can efficiently prevent AML cell egress from the bone marrow into the circulation.

An embodiment of the invention, shows that elastase inhibition inhibits AML proliferation, AML migration and AML egress from the BM. Therefore, elastase inhibition can be used to treat AML.

We showed a differential effect of elastase in homing and growth of AML and normal hematopoietic stem and progeniotr cells, which can be advantageously used for obtaining a population of Hematopoietic stem cells substantially free of AML cells from a leukemic patient. For example, a mixture of normal hematopoietic stem cells and leukemic, cells, extracted from a patient, could be grown in a medium supplemented with an elastase inhibitor, and fast migrating, optionally in an SDF-1 gradient, cells comprised of a population of cells enriched with normal hematopoietic stem cells can be collected.

We also showed that elastase in the bone marrow of AML patients correlated with the maturation stage of the leukemic cells e.g. the highest concentration of BM elastase was found in M3 and M4 AML subtypes and a very low level in the bone marrow of undifferentiated M0 AML, therefore, monitoring the level of elastase in the BM of leukemic patients could be used to diagnose the maturation stage of AML cells in a patient e.g. as shown e.g. in example 1.

The term "inhibitor of elastase" within the context of this invention refers to any molecule modulating elastase production and/or action in such a way that elastase production and/or action is attenuated, reduced, or partially, substantially or completely prevented or blocked. The term "elastase inhibitor" is meant to encompass inhibitors of elastase production as well as of inhibitors of elastase action. Elastase inhibitors can be small molecules or polypeptides or peptides.

An inhibitor of production can be any molecule negatively affecting the synthesis, processing or maturation of elastase. The inhibitors considered according to the invention can be, for example, suppressors of gene expression of the elastase, antisense mRNAs reducing or preventing the transcription of the elastase mRNA or leading to degradation of the mRNA, proteins impairing correct folding, or partially or substantially preventing secretion of elastase, proteases degrading elastase, once it has been synthesized, inhibitors of elastase activation and inhibitors of elastase secretion from granules to the scell surface. An inhibitor of elastase action can be a natural inhibitor such as a1 anti trypsin. Antagonists of elastase can either bind to or sequester the elastase molecule itself with sufficient affinity and specificity to partially or substantially neutralize the elastase or elastase binding site(s) responsible for elastase binding to its ligands .

Inhibitors of elastase action may also be elastase antibodies, such as polyclonal or monoclonal antibodies, or any other agent or molecule preventing the binding of elastase to its targets, thus, diminishing or preventing reactions mediated by elastase.

In a preferred embodiment of the present invention, the inhibitor of elastase is selected from EI, a1 anti trypsin antibodies directed against elastase, antagonists of elastase which compete with elastase, and elastase binding proteins.

Within the context of the present invention, the expressions "migration" and "homing" are used synonymously.

The hematopoietic human stem and/or precursor cells to be used according to the invention can be embryonic and/or neonatal such as human cord blood cells and/or adult stem cells (e.g. bone marrow, mobilized peripheral blood cells as described ( Kollet et al. 2001). The source of stem and/or precursor cells may be allogeneic (such as HLA-mismatched donors), preferably syngeneic (such as HLA-matched siblings), and most preferably autologous (i.e. derived from the own patient).

Stem cells and/or progenitor cells can be collected and isolated from peripheral blood of a donor or the patient treated with a mobilization inducing agent such as G-CSF or from the bone marrow by chirurgic intervention. G-CSF induces mobilization of stem cells and/or progenitor cells from hematopoietic organs e.g. bone marrow to the peripheral blood.

Hematopoietic stem and progenitor cells are isolated from their cellular mixtures with mature blood cells in said hematopoietic sources by standard techniques (Kollet et al. 2001), e.g. the blood samples are diluted 1:1 in phosphate buffered saline (PBS) without Mg⁺²/Ca⁺². Low-density mononuclear cells are collected after standard separation on Ficoll-Paque (Pharmacia Biotech, Uppsala, Sweden) and washed in PBS. CD34⁺ cells can be purified, using the MACS cell isolation kit and MidiMacs columns or AutoMACS (Miltenyi Biotec, Bergisch Gladbach, Germany) according to the manufacturer's instructions purity of more than 95% can be obtained. Isolated CD34⁺ cells can be either used immediately for homing experiments or after overnight incubation with RPMI supplemented with 10% fetal calf serum (FCS) or serum free and stem cell factor (SCF) (50 ng/mL). Various techniques can be employed to separate the cells by initially removing cells of dedicated lineage. Antibodies recognising a marker of a specific lineage can be used for separation of the required cells, for example antibodies to the CXCR4 receptor. Also, enriched CD34⁺ cells can be further labeled with human specific monoclonal antibody (mAb) anti-CD34 FITC (Becton Dickinson, San Jose, CA) and anti-CD38 PE (Becton Dickinson, San Jose, CA) and sorted for CD34⁺CD38^{-/low}- or CD34⁺CD38⁺-purified subpopulations by FACSVentage (Becton Dickinson); purity of 97% to 99% may be obtained.

Various techniques of different efficacy can be used to obtain enriched preparations of cells. Such enriched preparations of cells are up to 10%, usually not more than 5%, preferably not more than about 1%, of the total cells.

Procedures for separation of HSC/progenitor cell lineages comprise physical separation e.g. density gradient centrifugation, cell surface (lectin and antibody affinity), magnetic separation etc. A preferred technique that provides good separation is flow cytometry.

Methods of determining the presence or absence of a cell surface marker are well known in the art (Encyclopedia of Immunology Ed. Roitt. Delves, Vol-1 134). Typically, a labelled antibody specific to the marker is used to identify the cell population. Reagents specific for the human cell surface markers Thy-1 and CD34 are known in the art and are commercially available.

Methods for mobilizing stem cells into the peripheral blood are known in the art and generally involve treatment with a chemotherapeutic drug e.g. cyclophosphamide (CY) and cytokines e.g. G-CSF, GM-CSF, G-CSF+ IL3 etc.

Isolated patient's hematopoietic stem cells mobilized by cytokine stimulation with and without chemotherapy treatnment can be treated ex-vivo prior to transplantation, according to the invention, with elastase inhibitor to support survival and growth of homing competent hematopoietic stem cells and to inhibit proliferation/growth of leukemic cells.

Genetically modified HSC producing an elastase-inhibiting agent may be used according to the method of the invention. Gene transfer to HSC and/or precursors can be carried out by transduction of adeno-associated viruses, retroviruses, lentiviruses and adeno-retroviral chimera, encoding the therapeutic agent e.g. Elastase inhibitor, as described by Zheng et al. 2000 and Lotti et al. 2002. Such genetically modified HSC could be used according to the invention in leukemic patients.

The use of a vector for inducing and/or enhancing the endogenous production of an elastase inhibitor is also contemplated according to the invention. The vector may comprise regulatory sequences functional in the cells desired to express endogenous elastase inhibitor. Such regulatory sequences may be promoters or enhancers. The regulatory sequence may then be introduced into the right locus of the genome by homologous recombination, thus, operably linking the regulatory sequence with the gene, the expression of which is required to be induced or enhanced. This overexpression can be stable or transient. The technology is usually referred to as "endogenous gene activation" (EGA), and it is described e.g. in WO 91/09955.

In additon to natural elastase inhibitors such as a1 anti trypsin, inhibition of SDF-1 or its receptor CXCR4, which induces elastase activation in malignant human AML cells, can also be used (see Example 7). SDF-1 and CXCR4 inhibitors, which will also prevent elastase activation on AML cells are, for example, anti SDF-1 and anti CXCR4 antibody, AMD 3100, TC 140 and or any other inhibitor of this chemokine and receptor including proteolytic enzymes which inactivate the ligand and or the receptor (CD26 MMP2/9, cathepsin G).

An elastase inhibitor can be administered to a patient suffering of leukemia to reduce the leukemic load by preventing malignant cell proliferation. An elastase inhibitor in a combination with a mobilization agent could be administrated to a patient suffering of leukemia prior during or after HSC and/or progenitor transplantation wherein the transplantation is autologous or heterologous.

The present disclosure also relates to pharmaceutical compositions prepared for administration of an elastase inhibitor such as EI, a1-antitrypsin inhibitor, anti elastase antibody, SDF-1 inhibitor, CXCR4 inhibitor or a mixture of inhibitors by mixing the inhibitor, with physiologically acceptable carriers, and/or stabilizers and/or excipients, and prepared in dosage form, e.g., by lyophilization in dosage vials.

The invention further relates to pharmaceutical compositions, particularly useful for preventing leukemic cell proliferation and/or egress from hematopoietic organs to the peripheral blood comprising a therapeutically effective amount of an elastase inhibitor.

The present invention further relates to pharmaceutical compositions comprising a pharmaceutically acceptable carrier and an elastase inhibitor e.g. EI or a mixture of inhibitors for the treatment of patients suffering of leukemia. Preferably the elastase inhibitor may be administered by direct injection into the patient before after or during cell mobilization.

Alternatively an endogenous elastase inhibitor may be induced preferable by the administration of agents inducing endogenous elastase inhibitor.

An elastase inhibitor, as described above is the preferred active ingredients of the pharmaceutical compositions.

The pharmaceutical compositions may comprise a pharmaceutically acceptable carrier, an elastase inhibitor such as EI, a1-antitrypsin inhibitor, anti elastase antibody, SDF-1 inhibitor, CXCR4 inhibitor or a mixture thereof, and optionally further including one or more mobilizing agent.

The definition of "pharmaceutically acceptable" is meant to encompass any carrier, which does not interfere with effectiveness of the biological activity of the active ingredient and that is not toxic to the host to which it is administered. For example, for parenteral administration, the active agent(s) may be formulated in a unit dosage form for injection in vehicles such as saline, dextrose solution, serum albumin and Ringer's solution.

The active ingredients of the pharmaceutical composition according to the invention can be administered to an individual in a variety of ways. A therapeutically efficacious route of administration can be used, for example absorption through epithelial or endothelial tissues or by gene therapy wherein a DNA molecule encoding the active agent is administered to the patient (e.g. via a vector) which causes the active agent to be expressed and secreted in vivo. In addition, elastase inhibitor(s) according to the invention can be administered together with other components of biologically active agents such as pharmaceutically acceptable surfactants, excipients, carriers, diluents and vehicles.

For parenteral (e.g. intravenous, intramuscular) administration, the active elastase inhibitor(s) can be formulated as a solution, suspension, emulsion or lyophilized powder in association with a pharmaceutically acceptable parenteral vehicle (e.g. water, saline, dextrose solution) and additives that maintain isotonicity (e.g. mannitol) or chemical stability (e.g. preservatives and buffers). The formulation is sterilized by commonly used techniques.

The bioavailability of the active elastase inhibitor(s) according to the invention can also be ameliorated by using conjugation procedures which increase the half-life of the molecule in the human body, for example linking the molecule to polyethylenglycol, as described in the PCT Patent Application WO 92/13095.

The therapeutically effective amounts of the active molecule will be a function of many variables, including the type of molecule used, any residual cytotoxic activity exhibited by the molecule, the route of administration, the clinical condition of the patient.

A "therapeutically effective amount" is such that when administered, the elastase inhibitor results in decreased egress of leukemic cells to the circulation and or decreased proliferation and/or decreased migration and/or homing. The dosage administered, as single or multiple doses, to an individual will vary depending upon a variety of factors, including the molecule pharmacokinetic properties, the route of administration, patient conditions and characteristics (sex, age, body weight, health, size), extent of symptoms, concurrent treatments, frequency of treatment and the effect desired. Adjustment and manipulation of established dosage ranges are well within the ability of those skilled in the art, as well as in vitro and in vivo methods of determining the effect of the molecule in an individual.

According to the invention, the elastase inhibitor e.g. EI, a1-antitrypsin inhibitor, anti elastase antibody, SDF-1 inhibitor, CXCR4 inhibitor or a mixture thereof can be administered to an individual prior to, simultaneously or sequentially with other therapeutic regimens (e.g. multiple drug regimens) or agents, in a therapeutically effective amount, in particular with transplanted HSC and/or progenitor cells, and/or mobilization agents.

The disclosure further relates to a method of treating leukemia, comprising administering a pharmaceutically effective amount of an elastase inhibitor to a patient in need thereof.

Reference to known method steps, conventional methods steps, known methods or conventional methods is not any way an admission that any aspect, description or embodiment of the present invention is disclosed, taught or suggested in the relevant art.

The foregoing description of the specific embodiments will so fully reveal the general nature of the invention that others can, by applying knowledge within the skill of the art (including the contents of the references cited herein), readily modify and/or adapt for various application such specific embodiments, without undue experimentation.

Therefore, such adaptations and modifications are intended to be within the meaning a range of equivalents of the disclosed embodiments, based on the teaching and guidance presented herein.

### EXAMPLES

**Example 1: Primary human AML cells secrete abnormal high amount of elastase** t**hat correlate with the blasts levels in the circulation.** Secreted elastase in the bone marrow (BM) and peripheral blood (PB) plasma was quantitated by ELISA (according to the manufacturer's instructions, Bender MedSystems, San Bruno, CA) in 10 AML patients having different FAB (French-American-British classification, see table A above) subtypes. We found a variable amount of elastase in the BM and PB among samples (Fig: 1A). Interestingly, a correlation between the level of elastase in the BM and the subtypes (or FAB) of AML could be observed. For example, the poorly differentiated AML type (M0) had very low levels of elastase in the bone marrow, while more differentiated cells had a higher levels of the enzyme. APL and myelomonocytic leukemia (M4) had a remarkably high amount of elastase secreted in the BM (Fig. 1A). However, no correlation was found between variable levels of elastase in the PB and the different AML subtypes. When compared to PB plasma of normal individuals, a significantly higher concentration of elastase was observed in most AML patients (Fig. 1B). Of note, in two AML patients (M2 and M5) that had almost no blast cells in the circulation, normal levels of elastase were detected (Fig. 1B). This observation suggests that the concentration of PB elastase may correlate with the number of circulating leukemic blasts. Indeed when we compared the amount of secreted elastase protein in the plasma and the number of AML blast cells in the peripheral blood, a significant correlation between these parameters was found (Fig. 1C). These results demonstrate that the level of secreted elastase protein in the plasma is due to AML blast cells present in the blood and suggest that elastase may participate in malignant AML cell dissemination. Noteworthly, elastase concentration was very low in the PB plasma from a M0 type patient despite high white blood cell counts (WBC).

**Example 2: AML cells constitutively express elastase on their cell surface.** In neutrophils, elastase expression is induced after neutrophil activation or during degranulation occurring when the cells die. Elastase can be found in the conditioned medium of activated neutrophils. However, activated neutrophils express also membrane bound elastase, which, upon a chemotactic signal, is re-localized on the leading edge of migrating neutrophils (Cepinskas, 1999). Since AML cells displayed deregulated activation of elastase expression, we explored the expression level and localization of elastase in 4 AML cell lines (HL-60, U937, ML-1, ML-2) as well as in primary AML cells.

Cell surface elastase on primary AML and cell lines or on normal CB CD34+ enriched cells was analyzed by flow cytometry as follows: AML cells were incubated with polyclonal rabbit anti-human elastase (Biodesign, Kennebunkport, ME) for 30 min., washed and antibody binding was detected using secondary anti-rabbit-FITC (Figure 2A upper panel and 2B).

Cytoplasmic elastase was detected as follows: by intracellular flow cytometry staining after fixation in 4% paraformaldehyde 20 min and permeabilization in 0.5% triton for 10 min (Fig 2A lower panel). Using this approach, we found high levels of elastase on the surface of AML cells. In addition to FACS, we confirmed the presence of membrane-bound elastase by immunofluoresence microscopy (Figure 2B).

In addition, we found no co-localization between CXCR4 and co-localization between CXCR4 and elastase (data not shown).

These results suggest that AML cells not only secrete elastase independently of external stimulus, but also constitutively express elastase homogeneously assembled on the cell surface. Cepinskas et al.( Journal of Cell Science 112, 1937, 1999) indicated that membrane bound elastase in activated neutrophils is catalytic active and resistant to proteolyses by circulating proteases. Thus, membrane bound elastase in AML cells may allow AML cells to penetrate the bone marrow endothelial barrier and facilitate their dissemination into the circulation.

**Example 3: Elastase inhibitor decreases SDF-1 induced migration of AML cells.** To study the role of elastase in migration of AML cells, we evaluated the effect of elastase inhibition on spontaneous, or SDF-1-induced AML migration by in vitro transwell assay. The migration assay was carried out as follows: a total of 600mL RPMI supplemented with 10% FCS in the presence or absence of 125 ng/mL recombinant human SDF-1 (rhSDF-1) (Peprotech, Rocky Hill, NJ) was added to the lower chamber of a Costar 24-well transwell plates with 5mm pore filters (Coming, NY). One hundred thousand primary MNC leukemic cells or AML cell lines with or without pretreatment for 30 min. with elastase inhibitor (EI) MeOSuc-AAPV-CMK (10 µg/ml Calbiochem, La jolla, CA) or monoclonal mouse anti human neutrophil elastase Ab (50-100 ul of 70 µg/ml, Dako, Glostrup, Denmark) were added to the upper chamber and were allowed to migrate to the lower chamber for 4 hours at 37°C. Migrating cells were collected from the lower chamber and counted using a FACSCalibur (Becton Dickinson).

The results obtained by the in vitro assay showed that pre-incubation of AML cells, primary and cell lines, for 30 min with 5-10 µg/ml EI significantly reduced SDF-1 dependent migration of the cells (p=0.039) (Fig. 3A). The spontaneous migration, in absence of SDF-1, was also reduced (data not shown). In order to confirm the role of elastase in AML migration, we used in the same assay, instead of EI, neutralizing anti-elastase Abs, or the protease inhibitor a1-antitrypsin inhibitor 100 µg/ml (figure 3B and C respectively). The results obtained with the antibody and with the protease inhibitor were similar to the results obtained with EI and confirmed the role of elastase in AML migration.

These observations demonstrate that elastase is important for both spontaneous and SDF-1 dependent AML migration. To test whether the decreased migration by elastase inhibition is eventually due to reduction in CXCR4 expression, we examined the effect of elastase inhibition on AML CXCR4 expression. We found that EI did not change the level of cell surface CXCR4 expression as examined by FACS (data not shown). Thus, it appears that the role of elastase in migration of AML cells is not working via CXCR4 regulation.

Cell migration requires cytoskeletal rearrangements, and SDF-1 was shown to induce cytoskeletal rearrangements, which lead to formation of protrusions and cell polarization (Avigdor et al Blood. 2004 Apr 15;103(8):2981-9. Epub 2004 Jan 15.). When leukemic cells were pre-treated with elastase inhibitor (EI), the formation of SDF-1 induced protrusions was prevented (Fig 3D ). Of note, basal cell polarization in absence of SDF-1 was also abolished by EI. These results suggest that elastase participates in leukemic cell motility through direct regulation of cytoskeletal rearrangements and cell polarization.

Since transwell assay was performed on bare filters, the effect observed is not due to degradation of extracellular matrix (ECM) macromolecules. Indeed, we demonstrated that elastase inhibition prevented cell polarization and protrusion formation, implying the regulation of cytoskeletal rearrangements by elastase.

The results show that inhibition of elastase by either elastase inhibitors, a1-antitrypsin or elastase-neutralizing ABs affected migration of AML cells, both spontaneous and SDF-1 induced. Thus, the results show that elastase is necessary for migration of AML cells.

**Example 4: Elastase is necessary for homing of human AML cells into the BM of NOD/SCID**. The pre-clinical immune-deficient NOD/SCID mice model allows engraftment of human AML stem cells and mimics many aspects of human AML (Lapidot Nature. 1994 Feb 17;367(6464):645-8.).

In order to assess the effect of elastase inhibition on the in vivo migration (or homing) of human AML cells to the BM, primary AML mononuclear cells (MNC) cells (5x10⁶) were injected into sublethally irradiated B2mnull NOD/SCID mice, either untreated or after 30 minute incubation with EI (10µg/ml).

More specifically, human primary AML MNC, enriched CD34+ AML or CB cells at the indicated cell doses were suspended in 500 ml RPMI with 10% FCS incubated for 30 minutes in 37°C either with or without elastase inhibitor (10µg/ml) before injection to mice via the dorsal tail vein. Mice were sacrificed 16 hours after transplantation, BM cells flushed from both femurs and tibias bones were harvested and resuspended into single-cell suspension. The percentage of human cells was determined by immunostaining with anti-human CD45-FITC mAB (Immuno Quality Products, Groningen, The Netherlands). Human Fc receptors were blocked with human plasma (1%) and murine Fc receptors by anti-mouse CD16-CD32 (Pharmingen) (1:50). Isotype control antibodies were used in order to exclude false positive cells (BD). After staining, the cells were analyzed on a FACS Calibur (BD) using Cell Quest software.

The results obtained show that homing of M2 and M4 primary AML cells into the NOD/SCID mice BM was significantly decreased in EI treated cells as compared to the untreated control cells (Fig. 4A/B). Interestingly, homing of primary M1 and M5 AML cells was not inhibited but rather increased (Fig. 4A).

We next examined the homing of normal enriched CD34+ cells after elastase inhibition. In order to analyze similar primitive stem cell populations, CB CD34+ cells were compared to primary enriched CD34+ AML cells of three other patients (1.5-3x10⁶) with different FAB subtypes. As with MNC cells from some patients, inhibition of primary enriched CD34+ AML cells was observed. Elastase inhibition decreasedhoming the homing of AML enriched human CD34+ cells to the murine BM (Fig. 4C), while homing of normal CD34+ cells was increased (Fig. 4C). Taken together, these results indicate that elastase has an opposite effect on homing of normal and myeloid leukemia CD34+ cells.

**Example 5: AML cell egress from the BM into the circulation is inhibited by administration of elastase inhibitor in vivo.** G-CSF induces egress of stem cells from the BM into the circulation (mobilization). Mobilization appears to be caused by severe changes in the BM hematopoietic microenvironment including degradation of VCAM-1, c-kit receptor and SDF-1 by proteolytic enzymes such as cathepsin G and elastase. [Petit, Nat Immunol. 2002 Jul;3(7):687-94, 2002;Levesque, 2003 J Clin Invest. 2003 Jan;111 (2):187-96, Levesque Exp Hematol. 2003 Feb;31(2):109-17.]. Blocking elastase prevents G-CSF induced mobilization (Petit and Lapidot Exp Hematol. 2002 Sep;30(9):973-81. 2002). Leukemic cells express high level of several proteases such as MMP-2, MMP-9, MT1-MMP (Ries et al Clinical cancer research 1999, (5) 1115) and elastase. In view of our finding that elastase has a role in regulation of AML cell motility and homing (Example 3 and 4) we assumed that, out of all the proteases expressed by AML cells, elastase may facilitate egress of AML cells from the BM into the circulation.

In order to test this assumption, primary AML cells (20-40x10⁶) were injected into sublethally irradiated NOD/SCID mice to establish human AML-mice chimerism and AML egress from the bone marrow into the circulation was monitored, two-four weeks later, in mice treated with elastase inhibitor as compared to non treated mice.

More specifically, human primary AML MNC cells (10-30x10⁶) or HL60 AML cell line (20x10⁶) was injected into NOD/SCID mice. Two-four weeks later, EI (1 mg) was injected once a day for 4 consecutive days. PB from mice asphyxiated with dry ice was collected by cardiac aspiration in heparinized tubes and BM was collected as mentioned above. Percentage of human cells was determined by immunofluorescence for CD45 as described above.

The results observed show that AML cell egress to the PB was decreased in EI treated mice as compared to untreated mice (Fig. 5 and table B). Similar results were observed with both, human primary cells and human AML line.

These results show for the first time that elastase participates in the regulation of human AML cell emigration from the BM into the circulation and that administration of elastase inhibitor can efficiently prevent AML cell egress from the bone marrow into the circulation.

**Table B**

| | | CTL | | | EI | |
|---|---|---|---|---|---|---|
| | BM (%) | PB (%) | PB/BM | BM (%) | PB (%) | PBBM |
| #1 M2 | 80 | 56 | 0.7 | 80 | 6 | 0.07 |
| #2 M4 | 35 | 5 | 0.14 | 50 | 2.5 | 0.05 |
| #3 M4 | 79 | 14 | 0.177 | 12.7 | 0.5 | 0.039 |
| #4 | 7.4 | 0.18 | 0.024 | 16.9 | 0.25 | 0.014 |
| HL-60 | 3.1 | 13.5 | 4.8 | 4 | 2.4 | 0.6 |

**Example 6: AML cell growth is elastase dependent.** AML is characterized by extensive and uncontrolled AML cell proliferation within the BM. We checked the possibility that elastase may affect AML cell proliferation.

To test the role of elastase in proliferation of AML cells, primary AML cells (1x10⁶/ml), AML cell lines (1x10⁴/ ml) or normal CD34+ enriched cord blood (CB) cells (1x10⁵/ml) were grown in RPMI supplemented with 10% FCS with or without elastase inhibitor (10µg/ml). The number of viable cells was determined on days 0, 1, 3, 5 and 7 using trypan blue exclusion.

The effect of EI on the proliferative rate of primary MNC AML cells and AML cell lines (ML-2, U937) was evaluated after 3-7 days respectively in culture. The number of primary cells was reduced after 3 days in culture, and addition of EI significantly decreased the number of viable AML cells (Fig 6A). After 7 days in culture the number of ML2 and U937 was increased and cell proliferation was inhibited by culturing cells in the presence of EI (Fig 6A). Thus, the results indicate that elastase induces proliferation of AML cells.

When normal CB CD34+ cells were cultured in the same conditions, elastase inhibition enhanced their proliferation rate (Fig 6A). Moreover, we found that the percentage of primitive progenitor cells CD34+/38- was significantly increased after 3 days in culture in the presence of cytokines and EI (Fig 6B). Thus, the results indicate that elastase may inhibit proliferation of normal cells and suggest that it has a role in differentiation of normal stem cells.

Therefore, it was found according to the present invention that elastase is necessary for AML proliferation and that elastase inhibition can efficiently prevent AML cell growth and, at the same time, can induce proliferation of normal CD34+ cell growth maintaining primitive transplantation-competent CD34+/38- stem cells in the culture.

**Example 7: SDF-1 increases expression of surface elastase on AML cells and decreases it in normal CD34+ cells.** We recently showed that malignant human AML and pre B ALL cell homing to the BM and spleen NOD/SCIDB2mnull mice is also dependent on SDF-1/CXCR4 interactions [Tavor Cancer Res. 2004 Apr 15;64(8):2817-24.]. We assumed that part of the effect of SDF-1 in migration of AML cells may be via regulation of elastase expression.

In order to confirm our assumption, primary AML cells and normal CB CD34+ cells were treated with SDF-1 (200ng/ml) for 1 and 3 hours, and expression of cell surface elastase was determined by FACS (Figure 7) .

The result obtained show that SDF-1 has opposite effect on cell surface elastase expression in AML versus elastase expression in normal CB CD34+ cells, while SDF-1 increase surface elastase in AML cells, it decreases surface elastase on normal CB CD34+cells.

Thus, in light of the results obtained, SDF-1 affects AML migration, in contrast to normal CD34+ cell migration, by upregulating cell surface elastase expression.

**Example 8: Cell cultures.** Cell lines: Human myeloid U937, HL60, ML2 and ML1 (Hadassah University Hospital, Jerusalem, Israel) were grown in RPMI with 10% fetal calf serum (FCS).

Human cells: human cord blood (CB) cells from full-term deliveries and peripheral blood (PB) and/or BM cells, from 15 newly diagnosed AML patients were obtained. The diagnosis of leukemia was based on routine morphologic evaluation, immunophenotyping and cytochemical smears using the FAB classification.

The samples were diluted 1:1 in phosphate-buffered-saline (PBS). Low-density mononuclear cells (MNC) were collected after standard separation on Ficoll-Paque (Pharmacia Biotech, Uppsala, Sweden), and washed in PBS. CD34+ cells were enriched using the MACS cell isolation kit and AutoMacs magnetic cell sorter (Miltenyi Biotech, Bergisch Gladbach, Germany) according to the manufacturer's instructions, obtaining purity of more than 95%. Cells were used fresh or frozen in FCS plus 10% dimethyl sulfoxide (DMSO) for storage in liquid nitrogen.

### REFERENCES

Aprikyan AA, Dale DC. Mutations in the neutrophil elastase gene in cyclic and congenital neutropenia. Curr Opin Immunol. 2001;13:535-538.
Ginzberg HH, Cherapanov V, Dong Q, et al. Neutrophil-mediated epithelial injury during transmigration: role of elastase. Am J Physiol Gastrointest Liver Physiol. 2001;281:G705-717
Bank U, Ansorge S. More than destructive: neutrophil-derived serine proteases in cytokine bioactivity control. J Leukoc Biol. 2001;69:197-206
Lee WL, Downey GP. Leukocyte elastase: physiological functions and role in acute lung injury. Am J Respir Crit Care Med. 2001;164:896-904
Bleul CC, Fuhlbrigge RC, Casasnovas JM, Aiuti A, Springer TA. 1996 "A highly efficacious lymphocyte chemoattractant, stromal cell-derived factor 1 (SDF-1)" J Exp Med 184, 1101-1109.
Drize N, Chertkov J, Samoilina N, Zander A. 1996 "Effect of cytokine treatment (granulocyte colony-stimulating factor and stem cell factor) on hematopoiesis and the circulating pool of hematopoietic stem cells in mice." Exp Hematol 24, 816-822.
Duhrsen U, Villeval JL, Boyd J, Kannourakis G, Morstyn G, Metcalf D. 1988 "Effects of recombinant human granulocyte colony-stimulating factor on hematopoietic progenitor cells in cancer patients." Blood 72,2074-2081.
El Ouriaghli F, Fujiwara H, Melenhorst JJ, et al. Neutrophil elastase enzymatically antagonizes the in vitro action of G-CSF: implications for the regulation of granulopoiesis. Blood. 2003;101:1752-1758
El-Ouriaghli F, Sloand E, Mainwaring L, et al. Clonal dominance of chronic myelogenous leukemia is associated with diminished sensitivity to the antiproliferative effects of neutrophil elastase. Blood. 2003;102:3786-3792
Grisham and Thorgeirsson, in Stem Cells, C.S. Ed. (Academic Press, San Diego, CA, 1997), chap, 8.
Heissig B, Hattori K, Dias S, et al. Recruitment of stem and progenitor cells from the bone marrow niche requires MMP-9 mediated release of kit-ligand. Cell. 2002;109:625-637
Hunter MG, Druhan LJ, Massullo PR, et al. Proteolytic cleavage of granulocyte colony-stimulating factor and its receptor by neutrophil elastase induces growth inhibition and decreased cell surface expression of the granulocyte colony-stimulating factor receptor. Am J Hematol. 2003;74:149-155
Kim and Broxmeyer 1998 "In vitro behavior of hematopoietic progenitor cells under the influence of chemoattractants: stromal cell-derived factor-1, steel factor, and the bone marrow environment." blood, 1, 100-110.
Kim CH, Broxmeyer HE. SLC/exodus2/6Ckine/TCA4 induces chemotaxis of hematopoietic progenitor cells: differential activity of ligands of CCR7, CXCR3, or CXCR4 in chemotaxis vs. suppression of progenitor proliferation. J Leuk Biol 1999; 66:455
Kollet O, Spiegel A, Peled A, Petit I, Byk T, Hershkoviz R, Guetta E, Barkai G, Nagler A, Lapidot T "Rapid and efficient homing of human CD34(+)CD38(-/low)CXCR4(+) stem and progenitor cells to the bone marrow and spleen of NOD/SCID and NOD/SCID/B2m(null) mice" 2001 Blood 97, 3283-91.
Lagasse et al. 2000 "Purified hematopoietic stem cells can differentiate into hepatocytes in vivo." Nature medicine 6, 1229-34.
Lagasse et al. 20001 "Toward regenerative medicine." Immunity 14, 425-36.
Lapidot 2001 Ann. NY Acad. Sci. "Mechanism of human stem cell migration and repopulation of NOD/SCID and B2mnull NOD/SCID mice. The role of SDF-1/CXCR4 interactions" 938 83-95
Lotti et al. Journal of Virology. 2002 "Transcriptional targeting of lentiviral vectors by long terminal repeat enhancer replacement." 76 (8) 3996-4007.
Mazo IB, von Andrian UH. 1999 "Adhesion and homing of blood-borne cells in bone marrow microvessels." Journal of leukocyte Biology 66,25-32.
Novelli, M. et al. 1996 "Polyclonal origin of colonic adenomas in an XO/XY patient with FAP" Science 272, 1187-1190.
Novikoff PM, Yam A, Oikawa I. "Blast-like cell compartment in carcinogen-induced proliferating bile ductule. " Am J Pathol 1996 May;148(5):1473-92.
Papayannopoulou T 1999 "Hematopoietic stem/progenitor cell mobilization. A continuing quest for etiologic mechanisms." Ann N Y Acad Sci 872, 187-197; discussion 197-9.
Peled A, Petit I, Kollet O, Magid M, Ponomaryov T, Byk T, Nagler A, Ben-Hur H, Many A, Shultz L, Lider O, Alon R, Zipori D, Lapidot T. 1999 "Dependence of human stem cell engraftment and repopulation of NOD/SCID mice on CXCR4." Science 283, 845-848.
Peled A, Grabovsky V, Habler L, Sandbank J, Arenzana-Seisdedos F, Petit I, Ben-Hur H, Lapidot T, Alon R 1999 "The chemokine SDF-1 stimulates integrin-mediated arrest of CD34(+) cells on vascular endothelium under shear flow." The Journal of Clinical Investigation, 104, 1199-1211.
Petersen et al. 1999 "Bone marrow as a potential source of hepatic oval cells" SCIENCE 284, 1168-70.
Papayannopoulou T. Current mechanistic scenarios in hematopoietic stem/progenitor cell mobilization. Blood. 2004;103:1580-1585. Epub 2003 Nov 1586.
Ponomaryov T, Peled A, Petit I, et al. "Induction of the chemokine stromal-derived factor-1 following DNA damage improves human stem cell function." J Clin Invest. 2000;106:1331-1339
Rosu-Myles M, Gallacher L, Murdoch B, Hess DA, Keeney M, Kelvin D, Dale L, Ferguson SS, Wu D, Fellows F, Bhatia M. 2000 "The human hematopoietic stem cell compartment is heterogeneous for CXCR4 expression." PNAS 97, 14626-14631. Siena S, Bregni M, Brando B, Ravagnani F, Bonadonna G, Gianni AM., 1989" Circulation of CD34+ hematopoietic stem cells in the peripheral blood of high-dose cyclophosphamide-treated patients: enhancement by intravenous recombinant human granulocyte-macrophage colony-stimulating factor." Blood 74,1905-1914.
Suzuki et al. Intern Immunol. 1998 "Loss of SDF-1 receptor expression during positive selection in the thymus" 10 8 1049-1056.
Sweeny, E.A., Priestley, G., Nakamoto, B., Papayannopoulou, T. 2000 "Sulfated Polysaccharides Increase Plevels of SDF-1 in Monkeys and Mice: Involvement in Mobilization of Stem/Progenitor Cells." Abstracts of the 42nd annual meeting of the American society of Haematology, December 1-5.
Tanaka et al. 1999 "Fetal microchimerism alone does not contribute to the induction of primary biliary cirrhosis" Hepatology 30, 833-838.
To LB, Haylock DN, Simmons PJ, et al. The biology and clinical uses of blood stem cells. Blood. 1997;89:2233-2258
To LB, Haylock DN, Simmons PJ, et al. The biology and clinical uses of blood stem cells. Blood. 1997;89:2233-2258
Wolfe et al. 1985 J Mol Biol. "Isolation and characterization of an alphoid centromeric repeat family from the human Y chromosome." 182, 477-485.
Zheng et al Nat Biotechnology 2000 "Genomic integration and gene expression by a modified adenoviral vector." 18, 176-180.

## Claims

1. The use of an elastase inhibitor in the manufacture of a medicament
for preventing or inhibiting egress of AML cells from the bone marrow to the blood in patients suffering of AML, or
for preventing or inhibiting proliferation of AML cells.

2. The use according to claim 1, for the treatment of acute leukemia.

3. The use according to claim 2, for the treatment of AML.

4. The use according to anyone of claims 1 to 3, wherein the inhibitor is an elastase-neutralizing antibody, or MeOSuc-AAPV-CMK, or a1-antitrypsin.

5. An elastase inhibitor for use in preventing or inhibiting egress of AML cells from the bone marrow to the blood in patients suffering of AML or for use in preventing or inhibiting proliferation of AML cells.

6. The elastase inhibitor of claim 5 for use in treating acute leukemia.

7. The elastase inhibitor of claim 6 for use in treating AML.

8. An elastase inhibitor according to anyone of claims 5 to 7, wherein the inhibitor is an elastase-neutralizing antibody, or MeOSuc-AAPV-CMK, or a1-antitrypsin,

## Patentansprüche

1. Die Verwendung eines Elastase-Inhibitors in der Herstellung eines Medikamentes
zur Prävention oder Inhibition des Austritts von AML-Zellen aus dem Knochenmark in das Blut bei Patienten, die unter akuter myeloischer Leukämie (AML) leiden, oder
zur Prävention oder Inhibition der Proliferation von AML-Zellen.

2. Die Verwendung gemäß Anspruch 1, zur Behandlung von akuter Leukämie.

3. Die Verwendung gemäß Anspruch 2, zur Behandlung von akuter myeloischer Leukämie (AML).

4. Die Verwendung gemäß eines beliebigen der Ansprüche 1 bis 3, wobei der Inhibitor ein Elastase-neutralisierender Antikörper, oder MeOSuc-AAPV-CMK, oder a1-Antitrypsin ist.

5. Ein Elastase-Inhibitor zur Verwendung in der Prävention oder Inhibition des Austritts von AML-Zellen aus dem Knochenmark in das Blut bei Patienten, die unter akuter myeloischer Leukämie (AML) leiden, oder zur Verwendung in der Prävention oder Inhibition der Proliferation von AML-Zellen.

6. Der Elastase-Inhibitor aus Anspruch 5 zur Verwendung in der Behandlung von akuter Leukämie.

7. Der Elastase-Inhibitor aus Anspruch 6 zur Verwendung in der Behandlung von akuter myelotischer Leukämie (AML).

8. Ein Elastase-Inhibitor gemäß einem beliebigen der Ansprüche 5 bis 7, wobei der Inhibitor ein Elastase-neutralisierender Antikörper, oder MeOSuc-AAPV-CMK, oder a1-Antitrypsin ist.

## Revendications

1. Utilisation d'un inhibiteur de l'élastase dans la fabrication d'un médicament destiné à empêcher ou inhiber la sortie des cellules de LMA hors de la moelle osseuse dans le sang de patients souffrant de LMA ou empêcher ou inhiber la prolifération des cellules de LMA.

2. Utilisation selon la revendication 1 destinée au traitement d'une leucémie aiguë.

3. Utilisation selon la revendication 2 destinée au traitement de la LMA.

4. Utilisation selon l'une quelconque des revendications 1 à 3, dans laquelle l'inhibiteur est un anticorps neutralisant l'élastase, ou MeOsuc-AAPV-CMK ou l'al-antitrypsine.

5. Inhibiteur de l'élastase destiné à être utilisé pour empêcher ou inhiber la sortie des cellules des LMA hors de la moelle osseuse dans le sang de patients souffrant de LMA ou destiné à être utilisé pour empêcher ou inhiber la prolifération des cellules de LMA.

6. Inhibiteur de l'élastase selon la revendication 5 destiné à être utilisé dans le traitement d'une leucémie aiguë.

7. Inhibiteur de l'élastase selon la revendication 6 destiné à être utilisé dans le traitement de la LMA.

8. Inhibiteur de l'élastase selon l'une quelconque des revendications 5 à 7, dans lequel l'inhibiteur est un anticorps neutralisant l'élastase, ou MeOsuc-AAPV-CMK ou l'alantitrypsine.
